# EUROPEAN PATENT SPECIFICATION

(11) **EP 2 217 093 B1**
(45) Date of publication and mention of the grant of the patent: **21.03.2012**
(21) Application number: 08843420.4
(22) Date of filing: 29.10.2008
(51) Int. Cl.: A23L 1/22, A23L 1/236

(54) **FLAVOUR-MODULATING COMPOUNDS**
GESCHMACKSMODULIERENDE VERBINDUNGEN
COMPOSÉS MODULANT L'ARÔME

(30) Priority: 29.10.2007 EP 07119479
(43) Date of publication of application: 18.08.2010
(73) Proprietor: Givaudan SA, 1214 Vernier (CH)
(72) Inventor: BOUTER, Nico, NL-1261 LK Blaricum (NL); KOENIG, Thorsten, NL-1411 GP Naarden (NL); VAN OMMEREN, Esther, NL-8241 AS Lelystad (NL); RENES, Harry, NL-8241 AS Lelystad (NL); WINKEL, Chris, NL-1402 GL Bussum (NL)
(74) Representative: McStea, John Anthony
(86) International application number: PCT/CH2008/000454
(87) International publication number: WO 2009/055953

(56) References cited:
- EP-A- 0 125 049
- EP-A- 0 189 938
- EP-A- 1 621 194
- WO-A-00/69282
- WO-A-02/100192
- WO-A-2007/002112
- WO-A-2007/033064
- WO-A-2007/084185
- WO-A-2007/096643
- US-A- 4 339 602
- US-A- 4 627 987
- US-A- 5 004 754
- US-A- 5 336 513
- US-B1- 6 287 620
- DATABASE WPI Week 200037 Thomson Scientific, London, GB; AN 2000-426093 XP002510468 & JP 2000 143581 A (EISAI CO LTD) 23 May 2000 (2000-05-23)
- DATABASE WPI Week 197909 Thomson Scientific, London, GB; AN 1979-16902B XP002510469 & JP 54 008731 A (SUN STAR HAMIGAKI) 23 January 1979 (1979-01-23)
- DATABASE WPI Week 200362 Thomson Scientific, London, GB; AN 2003-649114 XP002510470 & JP 2003 104932 A (MITSUBISHI RAYON CO LTD) 9 April 2003 (2003-04-09)

## Description

This disclosure relates to the field of flavour modulation in foodstuffs, beverages, tobacco products, oral care products and the like. In particular, it relates to substances that are capable of improving, especially enhancing and complementing, sweet flavour impact of other flavour and/or taste imparting substances present in the aforementioned products. Thus flavour-modulating substances are provided, as well as compositions containing said substances, methods employing said substances and uses of these substances for improving the flavour of foodstuffs, beverages, tobacco products and the like.

The flavour of foodstuffs and beverages consists of two parts: the aroma and the taste. In general what is perceived through the olfactory epithelium in the nasal cavity is referred to as 'aroma', whereas the term 'taste' is generally used to describe the sensory impact that is perceived via the mouth, especially the tongue. The flavour sensation experienced upon consumption, especially taste, provides the final analysis of food prior to ingestion thereof. Visual and olfactory (smell) signals already give a first indication but only after intake of the food into the mouth the final decision is made either to ingest or to reject the food. Sweet taste is usually a signal that the food is safe (appetising) leading to ingestion of the food. The 'reactions' to salt and umami are really dependent on the strength of the signal. Bitter and sour are usually repulsive taste sensations, leading to rejection. Temperature is another measure by which the food is judged just as well as aching sensations caused by substances such as capsaicin (hot pepper) and certain chemicals (such as carbon dioxide).

It has now been found that certain substances have the effect of modulating the flavour of foodstuffs, beverages, tobacco products and oral care products, especially for enhancing sweet taste impact of other flavour and/or taste imparting substances comprised in such products.

There is therefore provided a method of modulating the flavour impact of flavour- and/or taste-imparting substances in a product selected from the group of foodstuffs, beverages, oral care products and tobacco products, wherein the product does not comprise a sweetening effective amount of N-[N-(3,3-dimethylbutyl)-L-α-aspartyl]-L-phenylalanine methyl ester, comprising the addition thereto of at least one compound selected from the group consisting of compounds of the formula (I): 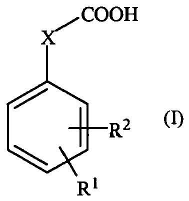 and edible salts and edible esters thereof, wherein -X- is selected from the group consisting of -CHOH-; -CO-;-R¹ and R² are independently selected from hydrogen, hydroxyl and lower alkoxyl; or R¹ and R² together form a 4-, 5- or 6-membered heterocyclic moiety comprising at least 1 heteroatom selected from oxygen and sulphur; wherein at least one of X, R¹ and R² comprises one or more carbon atoms.

By "modulating the flavour" is meant the complementing and modifying of the sensory impact. In other words, the substances hereinabove described and compositions in which they are included are capable of altering the taste and/or aroma impact of other flavour- and/or taste-imparting, substances present within the same product, with the proviso that this change in taste impact is not caused by the flavour contribution of said composition or substance *per se,* but instead that it mainly results from the combined effect of, on the one hand, the present flavour-modulating composition or substance, and on the other hand, the other flavour- and/or taste-imparting substance(s). The present flavour-modulating substances combine the capability of modulating the taste and/or aroma of other, flavour- and/or taste-imparting substances and a taste contribution of their own. The favourable impact of the present flavour-modulating substances is believed to be the result of the combination of these two effects.

Throughout this document, the term "flavour" is used to describe the sensory impact that is perceived via the mouth, especially the tongue, and the olfactory epithelium in the nasal cavity.

The term "lower" as used herein in connection to "alkoxyl" means that the moiety concerned comprises a carbon chain portion of not more than six carbon atoms, particularly of not more than four carbon atoms, and more particularly of not more than two carbon atoms. In a further particular embodiment, the lower alkoxyl is a branched or unbranched saturated C₁-C₆ alkoxyl, particularly C₁-C₄ alkoxyl, more particularly C₁-C₂ alkoxyl.

The term 'edible esters thereof', as used herein, refers to a derivative of a flavour-modulating substance of the invention and an acid or alcohol, formed by reaction of said acid or alcohol with a hydroxyl group or carboxyl group, respectively, that is present in said flavour-modulating substance, said derivative being suitable for human consumption, i.e. being non-toxic, and having flavour-modulating properties in accordance with the previous description. One group of edible esters of particular interest include those wherein the carboxylic acid group of the substance of formula (I) has been derivatized with a primary or secondary branched or unbranched lower alkanol, particularly a branched or unbranched staturated primary or secondary C₁-C₆ alkanol, more particularly a C₁-C₄ alkanol, most particularly methanol or ethanol.

The term "edible salt", as used herein, refers to a salt that is generally considered suitable for human consumption, particularly a non-toxic salt. Acceptable salts include base addition salts and acid addition salts of the corresponding free acid. These salts typically may be prepared by conventional means from the free acid of the present flavour-modulating substances. Illustrative base addition salts include metallic salts and organic salts. Metallic salts include alkali metal salts, such as sodium and potassium salts, alkaline earth metal salts, such as calcium and magnesium salts. Organic salts include salts made from secondary, tertiary and quaternary amines such as diethylamine, triethylamine, ethanolamine, diethanolamine, and salts made from cationic amino acids such as arginine, lysine and histidine. Examples of suitable acid addition salts include hydrochloride, phosphate, hydrogen phosphate, acetate, aspartate, ascorbate, citrate, gluconate, lactate, succinate, tartrate, etc.

The term "comprising" whenever used in this document is intended to indicate the presence of stated features, integers, steps, components, but not to preclude the presence or addition of one or more other features, integers, steps, components or groups thereof.

These flavour-modulating substances may be used to impart desirable taste attributes in a wide variety of applications and products. In addition, the present flavour-modulating substances are capable of modulating the taste and/or aroma impact of other flavour and/or taste imparting substances contained within these same products, thereby improving the overall flavour quality of these products.

Some of the compounds of Formula I are known compounds, but none are known as flavour modulators.

For example, it is known that anisic acid and cinnamic acid, both of which fall within the scope of Formula I, are substances that are abundant in certain natural materials as well as in extracts thereof that have traditionally been used for their specific flavour characteristics. However, it has never been suggested that these substances can in any way enhance the flavour impact or flavour perception of other flavour and/or taste imparting substances.

EP 0 125 049 discloses sweetness inhibitors which can be used to reduce the sweetness of sucrose, especially where it is not being used for its sweetening power but e.g. as a structural ingredient, for its antimicrobial action or as a carbohydrate source. Some of the sweetness inhibitors disclosed in EP 0 125 049 structurally resemble the substances of the present invention; EP 0 125 049 specifically discloses the substance phenyl pyruvic acid and suggests it has utility as a sweetness inhibitor. EP 0 125 049 does actually exemplify the application of phenylpyruvic acid as a sweetness inhibitor in a food product or test composition. EP 0 125 049 does not mention or suggest any taste or flavour characteristic other than sweetness inhibition, of substances encompassed by or resembling the above formula.

WO 00/69282 discloses compositions comprising neotame (N-[N-(3,3-dimethylbutyl)-L-α-aspartyl]-L-phenylalanine methyl ester) in combination with a hydrophobic acid additive and/or salts of neotame and such a hydrophobic acid additive. According to WO 00/69282 any hydrophobic acid additive or neotame salt thereof will affect at least one characteristic of the taste of neotame in a product, although it is acknowledged by the inventors of WO 00/69282 that not all combinations will be effective in all products conceivable. Since the entire group of hydrophobic acid additives disclosed in WO 00/69282 does not bear any structural resemblance other than being hydrophobic and containing an acidic moiety, and since WO 00/69282 mainly refers to the dissolution rate and the solubility of neotame, it is clear to the skilled person that the invention of WO 00/69282 concerns modification of the physicochemical properties of neotame compositions. The specific teachings of WO 00/69282 would not lead the skilled person to the conclusion that any of the hydrophobic acid additives could be effective in combination with any other type of sweetener, let alone any other type of flavour and/or taste imparting substance. Moreover, WO 00/69282 does not provide any teachings or suggestions to the effect that any of the hydrophobic acid additives themselves actually interact with taste receptors in the epithelium of the oral cavity and/or with the olfactory epithelium of the nasal cavity such as to modify the perceived flavour impact of other flavour and/or taste imparting substances.

US Patent 6287620 discloses a method of enhancing sweetness by adding 2-oxo-3-phenyl-propanoic acid or 3-(4-hydroxyphenyl)-2-oxo-propanoic acid.

WO 02/100192 describes a method of enhancing sweetness by adding chlorogenic acid. WO 2007/084185 concerns a method of enhancing sweetness by the addition of 4-methoxysalicylic acid.

Cinnamic acid is used to enhance sweetness in WO 2007/033064.

Caffeic acid and sodium ferulate are described as enhancing sweetness in US Patent 5336513.

Thus, the present invention, for the first time, provides the use of substances represented by the aforementioned formula (I) for improving the flavour perception of flavour and/or taste imparting substances other than N-[N-(3,3-dimethylbutyl)-L-α-aspartyl]-L-phenylalanine methyl ester.

There is also provided a flavour composition comprising at least 0.5 wt% of one or more flavouring substances in addition to at least 10 ppm of one or more flavour-modulating substances selected from the group consisting of substances according to formula (I), as hereinabove defined; with the exception of phenylpyruvic acid, anisic acid and cinnamic acid and salts and esters thereof, and with the proviso that the flavour composition does not comprise a sweetening effective amount of N-[N-(3,3-dimethylbutyl)-L-α-aspartyl]-L-phenylalanine methyl ester

It has been found that the abovementioned flavour-modulating substances are very useful flavour ingredients which, particularly in the presence of other flavour and/or taste imparting substances, are capable of imparting highly appreciated taste sensations to the products in which they are incorporated, specifically "vanilla" and/or "sweet". Because of this, the present substances can be employed to improve the flavour, especially taste, including "mouthfeel" and "body", of foodstuffs, beverages, tobacco products and oral care products.

The flavour-modulating substances of this disclosure are capable of imparting highly desirable taste attributes. In addition, it has been found that the flavour-modulating substances according to the invention are capable of complementing and modifying the sensory impact of other, flavour- and/or taste-imparting substances, contained in the aforementioned products, e.g. by enhancing and modulating "sweet taste impact" and/or "umami taste effects" thereof, especially "sweet taste impact".

Because the flavour-modulating substances according to this disclosure are not particularly volatile, they do not produce a strong aroma impact, even though they can affect the aroma impact of other flavour-imparting substances. Here the term "aroma" refers to the aspect of flavour that is perceived through the olfactory epithelium. Because of this low volatility, it is believed that the advantageous properties of these substances are somehow associated with the interaction that these substances have on the sensory receptors located within the mouth.

It was found that particularly satisfying results were obtained with flavour-modulating substances as hereinabove described wherein, -X- represents -CHOH- or -CO-. In particular embodiments, -X- is -CHOH-.

Other particularly satisfying results were obtained with flavour-modulating substances in accordance with this disclosure, wherein R¹ represents a substituent that is attached to the phenyl ring in the *para* position, i.e. to carbon atom 4, relative to the -X-COOH moiety. According to a particular embodiment, R¹ represents hydrogen, *p-*hydroxy, *p*-methoxy, particularly *p*-hydroxy or *p*-methoxy, and more particularly *p-*methoxy.

In a further particular embodiment, R² represents hydrogen, hydroxyl, methoxy or ethoxy, more particularly hydrogen, hydroxyl or methoxy, more particularly hydrogen or hydroxyl, and most particularly hydroxyl. Furthermore, in a particular embodiment, R² is attached to the phenyl ring in the *meta*-position.

In a further embodiment, at least one of R¹ and R² comprises one or more carbon atoms. One group of substances in accordance with the invention of particular interest includes those wherein at least one of R¹ and R² comprises one or more carbon atoms.

In a further embodiment, R¹ and R² together with two carbon atoms of the phenyl ring form a 5-membered heterocycle comprising at least 1, preferably 2, oxygen atoms. Particularly, R¹ and R² together represent a moiety having the formula -O-CH₂-O-. In addition, it is particularly preferred that R¹ and R² be attached to neighbouring carbon atoms of the phenyl ring, preferably to carbon atoms numbered 3 and 4, the carbon atom substituted with the -X-COOH moiety being designated as carbon atom 1.

In a further particular embodiment, there are provided flavour-modulating substances selected from the group of of 4-hydroxy-3-methoxymandelic acid; 4-methoxymandelic acid, (4-hydroxy-3-methoxyphenyl)-oxo-acetic acid ethyl ester; (3-hydroxy-4-methoxyphenyl)-oxo-acetic acid ethyl ester; (4-hydroxy-3-methoxyphenyl)-oxo-acetic acid; edible salts thereof and edible esters thereof. Particular embodiments are flavour-modulating substances selected from the group of 4-methoxymandelic acid, 4-hydroxy-3-methoxymandelic acid, 4-hydroxy-3-methoxymandelic acid, edible salts thereof and edible esters thereof, most preferably from 4-hydroxy-3-methoxymandelic acid, edible salts thereof and edible esters thereof.

As specified above, the flavour compositions of this disclosure do not comprise a sweetening effective amount of N-[N-(3,3-dimethylbutyl)-L-α-aspartyl]-L-phenylalanine methyl ester. As used herein, "a sweetening effective amount" refers to an amount that upon dosing the flavour composition does not produce an increase in sweetness as compared to an otherwise identical flavour composition that is free of N-[N-(3,3-dimethylbutyl)-L-α-aspartyl]-L-phenylalanine methyl ester. Neotame's threshold sweetening level has been determined to be approximately 4 ppm. In a particularly preferred embodiment of the invention, the present flavour compositions therefore comprise less than 40 ppm of N-[N-(3,3-dimethylbutyl)-L-α-aspartyl]-L-phenylalanine methyl ester, more preferably less than 20 ppm, more preferably less than 4 ppm, most preferably, the flavour compositions of the invention do not contain N-[N-(3,3-dimethylbutyl)-L-α-aspartyl]-L-phenylalanine methyl ester.

In another particular embodiment, flavour compositions as hereinabove defined are provided, wherein the molar ratio of flavour-modulating substances according to this disclosure to N-[N-(3,3-dimethylbutyl)-L-α-aspartyl]-L-phenylalanine methyl ester is higher than 2, preferably it is higher than 3, more preferably it is higher than 5, more preferably it is higher than 10, most preferably it is higher than 50.

In a further particular embodiment, a flavour composition as hereinabove defined is provided, wherein at least one flavour-modulating substance is present in an amount of at least 10 ppm, more preferably at least 100 ppm, most preferably at least 1000 ppm.

Typically the flavour compositions of the present inventions comprise the one or more flavour-modulating substances in an amount of less than 100,000 ppm, more preferably of less than 10,000 ppm, most preferably less than 5,000 ppm.

The expression "ppm" as used herein refer to amounts expressed in parts per million, 1 ppm corresponding to 1 mg/kg. This expression is a common term in the art of flavours and fragrances and understood by the skilled person as having the meaning given here.

As used herein the term "flavouring substance" is meant to encompass any food grade substance that is capable of imparting a detectable flavour impact, typically at concentrations below 1 wt.%, more preferably below 0.1 wt.%. Suitable examples of flavouring substances include alcohols, aldehydes, ketones, esters, ethers, acetates, nitriles, terpene hydrocarbons, nitrogenous or sulphurous heterocyclic compounds and essential oils, and said flavouring substances can be of natural or synthetic origin. Many of these are listed in reference texts such as the book by S. Arctander, Perfume and Flavor Chemicals, 1969, Montclair, New Jersey, USA, or its more recent versions, or in other works of a similar nature, as well as in the abundant patent literature in the field of flavours. It will be clear to the skilled person that the type of flavouring substance added would entirely depend on the type of product to which the composition is added.

In a particular embodiment, the flavour compositions according to the invention comprises one or more flavouring substances in an amount of at least 0.5 wt%, preferably at least 1 wt%, most preferably at least 2 wt%, based on the total weight of the composition. Typically the amount of flavouring substances comprised in the present flavour compositions is below 10 wt%, preferably it is below 5 wt%.

Typically, in the present flavour composition the taste modulating substances and flavouring substances as defined herein before are employed in a weight ratio of less than 1:50, preferably less than 1:100, more preferably less than 1:1,000 and most preferably in a weight ratio of less than 1:10,000.

In a particular embodiment, the present flavour composition comprises sweet taste imparting substances, especially natural or artificial sweeteners. Suitable examples of natural sweeteners include sucrose, fructose, glucose, high fructose corn syrup, xylose, arabinose, and rhamnose, the sugar alcohols erythritol, xylitol, mannitol, sorbitol, and inositol. Suitable examples of artificial sweeteners include AceK, aspartame, sucralose, and saccharine. These sweet taste imparting substances may be included in any suitable amount. Since the sweet taste imparting effect of these substances will be enhanced by the presence of the flavour-modulating substances of the invention, said amounts may typically be lower than those conventionally applied. Typically an amount will be included which is equivalent in sweetness to at least 0.5 wt% of sucrose.

The flavour compositions according to this disclosure may suitably be prepared in the form of a liquid, a paste or a powder. In a particular embodiment, the flavour composition is a free flowing powder. Typically the present flavouring compositions further comprise at a flavour carrier material. As used herein, the term flavour "carrier material" encompasses a bulk material that is practically neutral from a flavour point of view, i.e. which does not significantly alter the organoleptic properties of flavouring ingredients. Said carrier may be a liquid or a solid. Suitable examples of liquid carriers include emulsifying systems, i.e. a solvent and a surfactant system, or a solvent commonly used in flavours. As suitable no-limiting examples of solvents commonly used in flavours, one can cite compounds such as propylene glycol, triacetine, triethyl citrate, ethanol, vegetable oils or terpenes. Examples of solid carriers include absorbing gums or polymers or encapsulating materials. Examples of such materials, for example, may comprise wall-forming and plasticizing materials, such as mono, di- or trisaccharides, natural or modified starches, hydrocolloids, cellulose derivatives, polyvinyl acetates, polyvinylalcohols, proteins or pectins, maltodextrins or materials cited in reference texts such as H. Scherz, Hydrokolloids: Stabilisatoren, Dickungs- und Gehermittel in Lebensmittel, Band 2 der Schriftenreihe Lebensmittelchemie, Lebensmittelqualitat, Behr's VerlagGmbH & Co., Hamburg, 1996.

Typical examples of flavour compositions according to this disclosure include dairy flavourings and sweet flavourings.

A further aspect of this disclosure relates to a product selected from the group of foodstuffs, beverages, oral care products and tobacco products, preferably from the group of foodstuffs and beverages, said product comprising a flavour-modulating amount of one or more of the present flavour-modulating substances selected from the group of substances represented by formula (I), edible salts thereof and edible esters thereof, wherein -X- is selected from -CHOH-, -CO-; R¹ and R² are independently selected from hydrogen, hydroxyl and lower alkoxyl; or R¹ and R² together form a 4, 5 or 6 membered heterocyclic moiety comprising at least 1 heteroatom selected from oxygen and sulphur; wherein at least one of X, R¹ and R² comprises one or more carbon atoms, with the proviso that said flavour-modulating substance is not phenylpyruvic acid, anisic acid, cinnamic acid or a salt or ester thereof and wherein said product does not comprise a sweetening effective amount of N-[N-(3,3-dimethylbutyl)-L-α-aspartyl]-L-phenylalanine methyl ester.

Typically, the present product comprises at least 0.01 ppm of one or more of the present flavour-modulating substances. More preferably, products of this disclosure comprise at least 0.1 ppm, more preferably at least 1 ppm, most preferably at least 5 ppm of one or more of the flavour-modulating substances according to formula (I) or edible salts or esters thereof. Typically, the aforementioned products preferably contain the flavour-modulating substances in a concentration of not more than 10,000 ppm, preferably of not more than 1,000 ppm, more preferably of not more than 500 ppm, most preferably of not more than 100 ppm. The precise level in which the present substances are incorporated depends on the nature of the flavour-modulating substance(s) and the nature of the product, as will be clear to the skilled person, and as will be illustrated in the examples.

As specified above, the products of this disclosure do not comprise a sweetening effective amount of N-[N-(3,3-dimethylbutyl)-L-α-aspartyl]-L-phenylalanine methyl ester. In a particular embodiment, the present flavour compositions comprise less than 4 ppm of N-[N-(3,3-dimethylbutyl)-L-α-aspartyl]-L-phenylalanine methyl ester, more preferably less than 2 ppm, more preferably less than 1 ppm, most preferably, the products of the invention do not contain N-[N-(3,3-dimethylbutyl)-L-α-aspartyl]-L-phenylalanine methyl ester.

In another embodiment, products as hereinabove defined are provided, wherein the molar ratio of flavour-modulating substances according to the invention to N-[N-(3,3-dimethylbutyl)-L-α-aspartyl]-L-phenylalanine methyl ester is higher than 2, preferably it is higher than 3, more preferably it is higher than 5, more preferably it is higher than 10, most preferably it is higher than 50.

In a particular embodiment, the present product comprises sweet taste imparting substances, especially natural or artificial sweeteners. Suitable examples of natural sweeteners include sucrose, fructose, glucose, high fructose corn syrup, xylose, arabinose, and rhamnose, the sugar alcohols erythritol, xylitol, mannitol, sorbitol, and inositol. Suitable examples of artificial sweeteners include AceK, aspartame, sucralose, and saccharine. These sweet taste imparting substances may be included in any suitable amount. Since the sweet taste imparting effect of these substances will be enhanced by the presence of the flavour-modulating substances of the invention, said amounts may typically be lower than those conventionally applied. Typically an amount will be included which is equivalent in sweetness to 0.5-7 wt% of sucrose.

Paticular examples of foodstuffs according to the present invention include dairy products, such as yoghurts and ice creams, confectionery products, pastry products, bread products, baker's products, cereal products, rice products, tapioca products, 'sago products, biscuit products, desert products, jellies, jams and the like.

Preferred examples of beverages according to the present invention include soft drinks, dairy drinks, fruit juices and alcoholic beverages.

The term 'tobacco products', as used herein, refers to any type of tobacco product for smoking applications as well as for non-smoking applications. It is furthermore noted that tobacco-like products are available for both smoking and non-smoking applications. The use of the present flavour-modulating substances in tobacco substitutes is also encompassed by the present invention.

Suitable examples of oral care products according to the present invention include toothpastes, mouthwashes, dental floss, anti-plaque and anti-gingivitis compositions.

Particularly, the product is selected from the group of foodstuffs and beverages.

A further aspect of this disclosure relates to a method of improving the flavour of a product selected from the group of foodstuffs, beverages, oral care products and tobacco products, said method comprising incorporating in said product a flavour-modulating amount of one or more of the present flavour-modulating substances selected from the group of substances represented by formula (I), edible salts thereof and edible esters thereof, wherein wherein -X- represents -CHOH-, -CO-; R¹ represents hydrogen, hydroxyl or lower alkoxyl; R² represents hydrogen, hydroxyl or lower alkoxyl; or R¹ and R² together form a 4, 5 or 6 membered heterocyclic moiety comprising at least 1 heteroatom selected from oxygen and sulphur; wherein at least one of X, R¹ and R² comprises one or more carbon atoms with the exception of phenylpyruvic acid, anisic acid, cinnamic acid and salts and esters thereof, wherein said product does not comprise a sweetening effective amount of N-[N-(3,3-dimethylbutyl)-L-α-aspartyl]-L-phenylalanine methyl ester.

Particular examples and embodiments of the flavour-modulating substances, the products and amounts in accordance with this aspect of the invention are as described here above in relation to the other aspects of the invention.

As mentioned hereinabove, the present flavour-modulating substances are particularly suitable for modulating, especially improving and/or complementing sweet taste impact. Hence a preferred embodiment of the invention relates to the abovementioned use for improving and/or complementing sweet taste impact.

Particular embodiments of the flavour-modulating substance of this aspect of the disclosure are in accordance with those described hereinabove in relation to the other aspects of the invention. Most preferably the novel flavour-modulating substances in accordance with the invention is 4-methoxymandelic acid.

It is within the skills of the trained professional to design synthesis routes to prepare any novel substance provided by the present invention. Different routes may be available for producing a given substances, some of which may be more convenient and efficient than others.

The present invention and its preferred embodiments are further illustrated in the following examples, which are not intended to limit the scope in any way.

### Examples

### Example 1

A yoghurt drink containing 2% fat was sweetened with 6% sugar (sucrose). To one part of the sweetened yoghurt drink 20 ppm of 4-hydroxy-3-methoxy-mandelic acid was added. The yoghurt flavoured in accordance with the present invention was tasted and compared with the reference by a group of experienced flavourists. The flavoured sample was described as:
- being more sweet than the reference sample; and
- being more vanilla-like than the reference sample.

### Example 2

A number of flavour-modulating substances in accordance with the present invention were prepared and incorporated in 'sweet' and/or 'salt' test solutions (containing, respectively, 5 % sucrose or 0.6 % NaCl + 0.03 % MSG). A panel of professional flavourists evaluated the sensory characteristics of these samples containing flavour-modulating substances as compared to control samples not containing any of said flavour-modulating substances. The results obtained are summarized in table 1 below.

| **Flavour-modulating substance** | **Sensory Effects** | |
|---|---|---|
| (4-Hydroxy-3-methoxyphenyl)-oxo-acetic acid ethyl ester | On sweet: sweet enhancing, dust, liquorice | 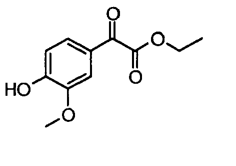 |
| | On salt: umami, liquorice | |
| (3-hydroxy-4-methoxy-phenyl)-oxo-acetic acid ethylester | On sweet: sweet enhancing, coumarine, caramellic, iodine, phenolic, fruity. Interesting for sweet, whisky fresh. | 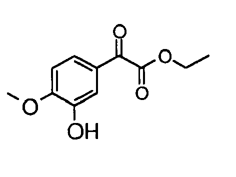 |
| | On salt: iodine, phenolic. | |
| (4-Hydroxy-3-methoxy-phenyl)-oxo-acetic acid | On sweet: sweet enhancing. | 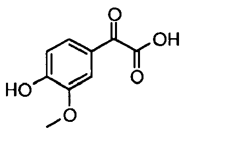 |
| 4-methoxymandelic acid | On sweet: very sweet, enhancing, sugar, slightly vanilla | 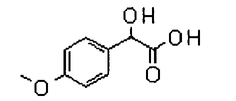 |

## Claims

1. A method of enhancing the flavour impact of flavour and/or taste imparting substances in a product selected from the group of foodstuffs, beverages, oral care products and tobacco products, wherein the product does not comprise a sweetening effective amount of N-[N-(3,3-dimethylbutyl)-L-α-aspartyl]-L-phenylalanine methyl ester, comprising the addition thereto of a compound selected from the group consisting of compounds of the formula (I): 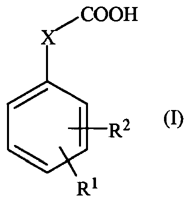 and edible salts and edible esters thereof, wherein -X- is selected from the group consisting of -CHOH-; -CO-; R¹ and R² are independently selected from hydrogen, hydroxyl and lower alkoxyl; or R¹ and R² together form a 4-, 5- or 6-membered heterocyclic moiety comprising at least 1 heteroatom selected from oxygen and sulphur; wherein at least one of X, R¹ and R² comprises one or more carbon atoms.

2. A method according to claim 1, wherein the product does not comprise N-[N-(3,3-dimethylbutyl)-L-α-aspartyl]-L-phenylalanine methyl ester.

3. A flavour composition comprising at least 0.5 wt% of at least one flavouring substance in addition to at least 10 ppm of at least one flavour-modulating substance selected from the group consisting of substances according to formula (I): 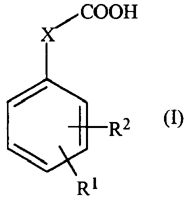 and edible salts and edible esters thereof, wherein -X- is selected from the group consisting of -CHOH-; -CO-;-R¹ and R² are independently selected from hydrogen, hydroxyl and lower alkoxyl; or R¹ and R² together form a 4-, 5- or 6-membered heterocyclic moiety comprising at least 1 heteroatom selected from oxygen and sulphur; wherein at least one of X, R¹ and R² comprises one or more carbon atoms; and with the proviso that the flavour composition does not comprise a sweetening effective amount of N-[N-(3,3-dimethylbutyl)-L-α-aspartyl]-L-phenylalanine methyl ester.

4. Flavour composition according to claim 3, wherein the flavour composition does not comprise N-[N-(3,3-dimethylbutyl)-L-α-aspartyl]-L-phenylalanine methyl ester.

5. Flavour composition according to claim 3, wherein -X- represents -CHOH-.

6. Flavour composition according to claim 3, wherein R¹ is in the *para* position.

7. Flavour composition according to claim 3, wherein R¹ and R² are independently selected from hydrogen, hydroxyl, methoxyl and ethoxyl or wherein R¹ and R² together represent a moiety having the formula -O-CH₂-O-.

8. Flavour composition according to claim 3, wherein the flavour-modulating substance is selected from the group of 4-hydroxy-3-methoxymandelic acid; 4-methoxymandelic acid, 2-oxo-3-phenyl-propionic acid, (4-hydroxy-3-methoxyphenyl)-oxo-acetic acid ethyl ester; (3-hydroxy-4-methoxyphenyl)-oxo-acetic acid ethyl ester; (4-hydroxy-3-methoxyphenyl)-oxo-acetic acid; edible salts thereof and edible esters thereof.

9. Flavour composition according to claim 3, wherein said one or more flavour-modulating substances are present in an amount of at least 100 ppm.

10. Product selected from the group of foodstuffs, beverages, oral care products and tobacco products, said product comprising a flavour-modulating amount of a flavour composition according to claim 3, and wherein the product does not comprise a sweetening effective amount of N-[N-(3,3-dimethylbutyl)-L-α-aspartyl]-L-phenylalanine methyl ester.

11. Product according to claim 10, wherein said flavour-modulating amount is an amount of at least 0.01 ppm.

## Patentansprüche

1. Verfahren zum Verstärken der Aromawirkung von aroma- und/oder geschmacksverleihenden Stoffen in einem Produkt, ausgewählt aus der Gruppe von Nahrungsmitteln, Getränken, Mundpflegeprodukten und Tabakprodukten, wobei das Produkt keine süßend wirksame Menge von N-[N-(3,3-Dimethylbutyl)-L-α-aspartyl]-L-phenylalaninmethylester umfasst, umfassend das Zugeben einer Verbindung dazu, ausgewählt aus der Gruppe bestehend aus Verbindungen der Formel (I): 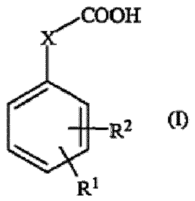 und genießbaren Salzen und genießbaren Estern davon, wobei -X- ausgewählt ist aus der Gruppe bestehend aus -CHOH-; -CO-; R¹ und R² unabhängig ausgewählt sind aus Wasserstoff, Hydroxy und Niederalkoxy; oder R¹ und R² zusammen eine 4-, 5- oder 6-gliedrige heterocyclische Einheit bilden, umfassend wenigstens 1 Heteroatom, ausgewählt aus Sauerstoff und Schwefel; wobei wenigstens eines von X, R¹ und R² ein oder mehrere Kohlenstoffatome umfasst.

2. Verfahren gemäß Anspruch 1, wobei das Produkt nicht N-[N-(3,3-Dimethylbutyl)-L-α-aspartyl]-L-phenylalaninmethylester umfasst.

3. Aromazusammensetzung, umfassend wenigstens 0,5 Gew.-% von wenigstens einem Aromastoff zusätzlich zu wenigstens 10 ppm des wenigstens einen aromamodulierenden Stoffs, ausgewählt aus der Gruppe bestehend aus Stoffen gemäß der Formel (I): 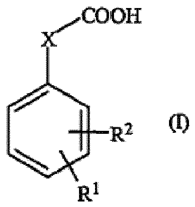 und genießbaren Salzen und genießbaren Estern davon, wobei -X- ausgewählt ist aus der Gruppe bestehend aus -CHOH-; -CO-; R¹ und R² unabhängig ausgewählt sind aus Wasserstoff, Hydroxy und Niederalkoxy; oder R¹ und R² zusammen eine 4-, 5- oder 6-gliedrige heterocyclische Einheit bilden, umfassend wenigstens 1 Heteroatom, ausgewählt aus Sauerstoff und Schwefel; wobei wenigstens eines von X, R¹ und R² ein oder mehrere Kohlenstoffatome umfasst; mit der Maßgabe, dass die Aromazusammensetzung keine süßend wirksame Menge von N-[N-(3,3-Dimethylbutyl)-L-α-aspartyl]-L-phenylalaninmethylester umfasst.

4. Aromazusammensetzung gemäß Anspruch 3, wobei die Aromazusammensetzung nicht N-[N-(3,3-Dimethylbutyl)-L-α-aspartyl]-L-phenylalaninmethylester umfasst.

5. Aromazusammensetzung gemäß Anspruch 3, wobei -X- -CHOH- darstellt.

6. Aromazusammensetzung gemäß Anspruch 3, wobei sich R¹ in der para-Position befindet.

7. Aromazusammensetzung gemäß Anspruch 3, wobei R¹ und R² unabhängig ausgewählt sind aus Wasserstoff, Hydroxy, Methoxy und Ethoxy oder wobei R¹ und R² zusammen eine Einheit mit der Formel -O-CH₂-O-darstellen.

8. Aromazusammensetzung gemäß Anspruch 3, wobei der aromamodulierende Stoff ausgewählt ist aus der Gruppe von 4-Hydroxy-3-methoxymandelsäure; 4-Methoxymandelsäure; 2-Oxo-3-phenylpropionsäure; (4-Hydroxy-3-methoxyphenyl)oxoessigsäureethylester; (3-Hydroxy-4-methoxyphenyl)oxoessigsäureethylester; (4-Hydroxy-3-methoxyphenyl)oxoessigsäure; genießbaren Salzen davon und genießbaren Estern davon.

9. Aromazusammensetzung gemäß Anspruch 3, wobei der eine oder die mehreren aromamodulierenden Stoffe in einer Menge von wenigstens 100 ppm vorhanden sind.

10. Produkt, ausgewählt aus der Gruppe von Nahrungsmittel, Getränken, Mundpflegeprodukten und Tabakprodukten, wobei das Produkt eine aromamodulierende Menge einer Aromazusammensetzung gemäß Anspruch 3 umfasst und wobei das Produkt keine süßend wirksame Menge von N-[N-(3,3-Dimethylbutyl)-L-α-aspartyl]-L-phenylalaninmethylester umfasst.

11. Produkt gemäß Anspruch 10, wobei die aromamodulierende Menge eine Menge von wenigstens 0,01 ppm ist.

## Revendications

1. Méthode pour augmenter l'impact aromatique de substances d'arome et/ou donnant du goût dans un produit choisi dans le groupe comprenant les aliments, les boissons, les produits d'hygiène buccale et les produits du tabac, le produit ne comprenant pas de quantité efficace édulcorante d'ester méthylique de N-[N-(3,3-diméthylbutyl)-L-α-aspartyl]-L-phénylalanine, comprenant l'addition à celui-ci d'un composé choisi dans le groupe constitué de composés de formule (1) : 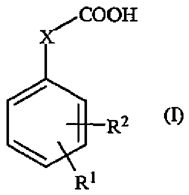 et de sels comestibles et d'esters comestibles de ceux-ci, dans laquelle -X- est choisi dans le groupe constitué de -CHOH- ; -CO- ; R¹ et R² sont choisis indépendamment parmi hydrogène, hydroxyle et alcoxyle inférieur ; ou R¹ et R² forment ensemble un motif hétérocyclique à 4, 5 ou 6 chaînons comprenant au moins 1 hétéroatome choisi parmi oxygène et soufre ; au moins l'un de X, R¹ et R² comprenant un ou plusieurs atomes de carbone.

2. Méthode selon la revendication 1, **caractérisée en ce que** le produit ne comprend pas d'ester méthylique de N-[N-(3,3-diméthylbutyl)-L-α-aspartyl]-L-phénylalanine.

3. Composition d'arome comprenant au moins 0,5 % en poids d'au moins une substance aromatisante outre au moins 10 ppm d'au moins une substance modulant l'arome, choisie dans le groupe constitué de substances selon la formule (1) : 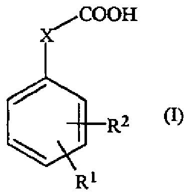 et de sels comestibles et d'esters comestibles de celles-ci, dans laquelle -X- est choisi dans le groupe constitué de -CHOH- ; -CO- ; R¹ et R² sont choisis indépendamment parmi hydrogène, hydroxyle et alcoxyle inférieur ; ou R¹ et R² forment ensemble un motif hétérocyclique à 4, 5 ou 6 chaînons comprenant au moins 1 hétéroatome choisi parmi oxygène et soufre ; au moins l'un de X, R¹ et R² comprenant un ou plusieurs atomes de carbone ; et à condition que la composition d'arome ne comprenne pas de quantité efficace édulcorante d'ester méthylique de N-[N-(3,3-diméthylbutyl)-L-α-aspartyl]-L-phénylalanine.

4. Composition d'arome selon la revendication 3, **caractérisée en ce que** la composition d'arome ne comprend pas d'ester méthylique de N-[N-(3,3-diméthylbutyl)-L-α-aspartyl]-L-phénylalanine.

5. Composition d'arome selon la revendication 3, **caractérisée en ce que** -X- représente -CHOH-.

6. Composition d'arome selon la revendication 3, **caractérisée en ce que** R¹ est en position para.

7. Composition d'arome selon la revendication 3, **caractérisée en ce que** R¹ et R² sont choisis indépendamment parmi hydrogène, hydroxyle, méthoxyle et éthoxyle ou **en ce que** R¹ et R² représentent ensemble un motif ayant la formule - O-CH₂-O-.

8. Composition d'arome selon la revendication 3, **caractérisée en ce que** la substance modulant l'arome est choisie dans le groupe constitué par l'acide 4-hydroxy-3-méthoxymandélique ; l'acide 4-méthoxymandélique, l'acide 2-oxo-3-phényl-propionique, l'ester éthylique d'acide (4-hydroxy-3-méthoxyphényl)-oxo-acétique ; l'ester éthylique d'acide (3-hydroxy-4-méthoxyphényl)-oxo-acétique ; l'acide (4-hydroxy-3-méthoxyphényl)-oxo-acétique ; les sels comestibles de ceux-ci et les esters comestibles de ceux-ci.

9. Composition d'arome selon la revendication 3, **caractérisée en ce qu'**une ou plusieurs substances modulant l'arome sont présentes dans une quantité d'au moins 100 ppm.

10. Produit choisi dans le groupe comprenant les aliments, les boissons, les produits d'hygiène buccale et les produits du tabac, ledit produit comprenant une quantité modulant l'arome d'une composition d'arome selon la revendication 3, et le produit ne comprenant pas de quantité efficace édulcorante d'ester méthylique de N-[N-(3,3-diméthylbutyl)-L-α-aspartyl]-L-phénylalanine.

11. Produit selon la revendication 10, **caractérisé en ce que** ladite quantité modulant l'arome est une quantité d'au moins 0,01 ppm.
